(19) Europäisches Patentamt
European Patent Office
Office européen des brevets

(11) Veröffentlichungsnummer: **0 483 063 B1**

# (12) EUROPÄISCHE PATENTSCHRIFT

(45) Veröffentlichungstag der Patentschrift: **07.09.94**

(51) Int. Cl.$^5$: **C07D 457/00**, A61K 31/48

(21) Anmeldenummer: **91810800.2**

(22) Anmeldetag: **17.10.91**

Die Akte enthält technische Angaben, die nach dem Eingang der Anmeldung eingereicht wurden und die nicht in dieser Patentschrift enthalten sind.

(54) **Ergolinderivate.**

(30) Priorität: **20.10.90 DE 4033496**

(43) Veröffentlichungstag der Anmeldung:
**29.04.92 Patentblatt 92/18**

(45) Bekanntmachung des Hinweises auf die Patenterteilung:
**07.09.94 Patentblatt 94/36**

(84) Benannte Vertragsstaaten:
**AT BE CH DE DK ES FR GB GR IT LI LU NL SE**

(56) Entgegenhaltungen:
**EP-A- 0 251 732**
**DE-A- 2 530 577**
**FR-A- 2 626 177**
**GB-A- 2 031 409**

(73) Patentinhaber: **SANDOZ LTD.**
**Lichtstrasse 35**
**CH-4002 Basel (CH)**
(84) Benannte Vertragsstaaten:
**BE CH DK ES FR GB GR IT LI LU NL SE**

(73) Patentinhaber: **SANDOZ-PATENT-GMBH**
**Humboldtstrasse 3**
**D-79539 Lörrach (DE)**
(84) Benannte Vertragsstaaten:
**DE**

(73) Patentinhaber: **SANDOZ-ERFINDUNGEN Verwaltungsgesellschaft m.b.H.**
**Brunner Strasse 59**
**A-1230 Wien (AT)**
(84) Benannte Vertragsstaaten:
**AT**

(72) Erfinder: **Amstutz, René**
**Talstrasse 394**
**CH-4204 Himmelried (CH)**
Erfinder: **Markstein, Rudolf**
**Ernst-Reuter-Strasse 2**
**W-7888 Rheinfelden (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Gegenstand der vorliegenden Erfindung sind neue Ergolinderivate, ihre Herstellung und Anwendung bei der therapeutischen Behandlung.

Diese im folgenden als neue Verbindungen bezeichneten Substanzen entsprechen insbesondere der Formel I,

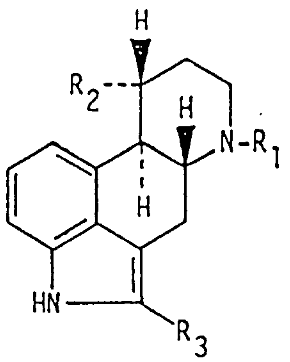

I

worin

$R_1$ Wasserstoff oder Alkyl mit 1 bis 4 Kohlenstoffatomen bedeutet,

$R_2$ für gegebenenfalls durch Fluor, Chlor, Brom, Alkyl oder Alkoxy mit je 1 bis 4 Kohlenstoffatomen oder Trifluoromethyl mono- oder disubstituiertes Phenyl steht und

$R_3$ Chlor, Brom oder Jod bedeutet,

in Form der freien Basen oder in Säureadditionssalzform.

Wie dies in der Fachliteratur üblich ist, werden mittels der obigen Darstellung der Formel I sowohl die Isomere mit der angegebenen Konfiguration wie auch ihre Antipoden umfasst. Die Erfindung betrifft beide Antipoden sowie ihre Gemische, beispielsweise die racemischen Gemische.

Eine Alkyl- oder Alkoxygruppe enthält vorzugsweise ein oder zwei Kohlenstoffatome und stellt insbesondere Methyl oder Methoxy dar.

$R_1$ bedeutet vorzugsweise Alkyl, insbesondere Methyl,

$R_2$ bedeutet vorzugsweise unsubstituiertes Phenyl,

$R_3$ steht vorzugsweise für Chlor.

Die bevorzugte Verbindung ist das (+)-[5R(5$\beta$,9$\alpha$,10$\alpha$)]-2-Chlor-6-methyl-9-phenyl-ergolin in Form der freien Base oder eines Säureadditionssalzes.

Erfindungsgemäss gelangt man zu den neuen Verbindungen und ihren Säureadditionssalzen, indem man Verbindungen der Formel II,

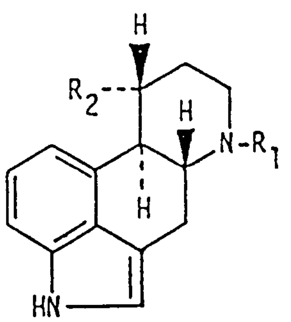

II

worin $R_1$ und $R_2$ obige Bedeutung besitzen, halogeniert und die erhaltenen Verbindungen der Formel I in Form der freien Basen oder in Säureadditionssalzform gewinnt.

Die Halogenierung der Verbindungen der Formel II kann nach an sich aus der Chemie der Mutterkornalkaloide bekannten Methoden erfolgen.

Die Aufarbeitung der erhaltenen Reaktionsgemische und die Reinigung der so gewonnenen Verbindungen der Formel I kann nach an sich bekannten Methoden durchgeführt werden.

Die Racemattrennung, falls erwünscht, kann auf an sich bekannter Weise erfolgen, z.B. via vorübergehende Säureadditionssalzbildung mit optisch aktiven Säuren, und fraktionierter Kristallisation der diastereomeren Säureadditionssalze. Optisch reine Verbindungen der Formel I können auch mittels optisch reinen Ausgangsverbindungen hergestellt werden.

Die freien Basen können in bekannter Weise in Säureadditionssalze umgewandelt werden und umgekehrt.

Die Ausgangsverbindungen der Formel II können nach dem folgenden Reaktionsschema, nach an sich bekannten Methoden, beispielsweise wie im nachfolgenden Beispiel 1 beschrieben, hergestellt werden:

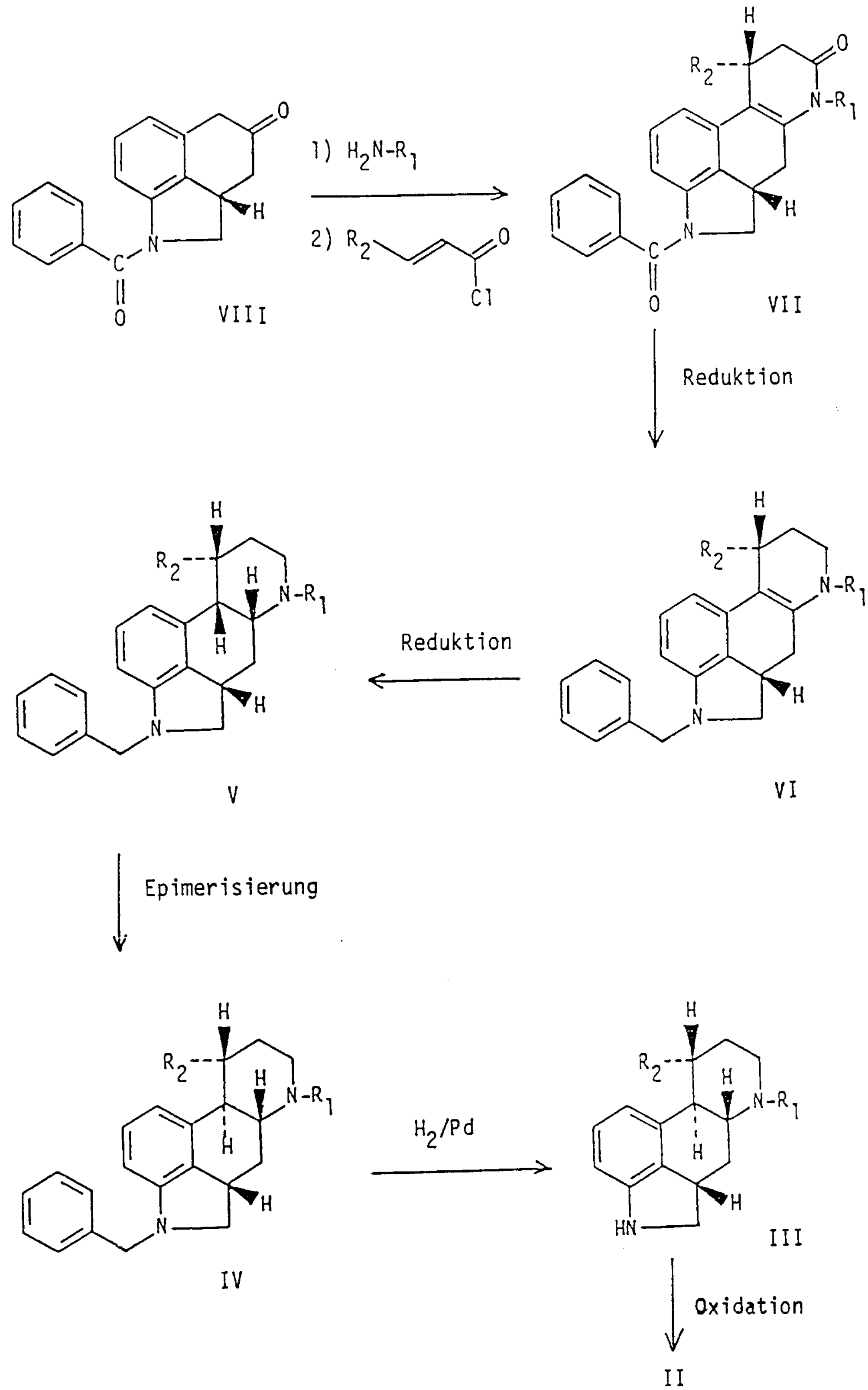


VIII VII VI V IV III II

Soweit die Herstellung der Ausgangsprodukte nicht beschrieben wird, sind diese bekannt oder nach an sich bekannten Verfahren herstellbar.

Die Verbindungen der Formel I und ihre physiologisch verträglichen Säureadditionssalze, im folgenden als erfindungsgemässe Verbindungen bezeichnet, sind in der Literatur bisher nicht beschrieben worden. Sie weisen im Tierversuch interessante pharmakodynamische Eigenschaften auf und können daher als Heilmittel verwendet werden.

Aus FR-A-2,626,177 sind analoge, in 2-Stellung unsubstituierte Ergolinderivate mit dopaminerger Wirkung bekannt. Aus DE-A-2,530,577 sind sowohl 2-unsubstituierte wie auch 2-halogenierte Ergolinderivate mit dopaminerger Wirkung bekannt. Aus GB-A-2,031,409 sind sowohl 2-unsubstituierte wie auch 2-halogenierte Isochinolinderivate mit neuroleptischer Wirkung bekannt. EP-A-0,251,732 beschreibt ein Herstellungsverfahren von 2-halogenierten Ergolinderivaten.

Im Gegensatz zu den unsubstituierten Verbindungen der o.e. FR-Anmeldung zeigen die erfindungsgemässen Verbindungen besonders eine Dopamin-antagonistische Wirkung in vivo am zentralen Nervensystem, was in den folgenden Tiermodellen nachgewiesen wird:
Bei sich frei bewegenden Ratten mit Gehirn-Dialyse [Methode nach A. Imperato und G. Di Chiara, European Journal of Pharmacology, 156 (1988) 385 - 393] wird mit Dosen von ca. 1 bis 5 mg/kg i.p. eine signifikante Zunahme der Dopamin-Freisetzung festgestellt.

Mit Dosen von ca. 2 mg/kg s.c. oder p.o. wird bei der Maus eine signifikante Hemmung des nach subkutaner Verabreichung von 0,5 mg/kg Apomorphin hervorgerufenen Aufrichtens beobachtet.

Mit Dosen von ca. 1 bis 10 mg/kg p.o. oder s.c. hemmen die erfindungsgemässen Verbindungen die nach Verabreichung von 10 mg/kg p.o. des $D_1$-Agonisten (-)-(6aR,12bR)-4,6,6a-7,8,12b-Hexahydro-7-methylindolo[4,3-ab]phenanthridin hervorgerufene lokomotorische Aktivitätssteigerung bei der Ratte. In diesem Versuch wird die zu prüfende Substanz eine Stunde vor dem $D_1$-Agonisten appliziert und die motorische Aktivität bei Rotlicht mittels Fotozellen gemessen.

Die erfindungsgemässen Verbindungen sind deshalb brauchbar als Dopamin-Antagonisten, z.B. zur Behandlung der Schizophrenie.

Eine empfohlene tägliche Dosis liegt im Bereich von ca. 10 bis 200 mg, insbesondere ca. 40 bis 160 mg, der erfindungsgemässen Verbindung. Geeignete Dosierungsformen für z.B. orale Anwendungen enthalten im allgemeinen ungefähr 2,5 bis 100 mg wirksame Substanz neben festen oder flüssigen Trägersubstanzen oder Verdünnungsmitteln.

Als Heilmittel können die erfindungsgemässen Verbindungen allein oder in geeigneter Arzneiform mit pharmakologisch indifferenten Stoffen verabreicht werden.

Gegenstand der Erfindung sind auch pharmazeutische Zusammensetzungen, die die erfindungsgemässen Verbindungen als Wirkstoffe enthalten. Für ihre Herstellung können die in der Pharmazie gebräuchlichen Hilfs- und Trägerstoffe verwendet werden. Geeignete galenische Formen sind z.B. Tabletten, Kapseln und Tropflösungen.

Die nachfolgenden Beispiele erläutern die Erfindung. Temperaturen erfolgen in Celsiusgraden und sind unkorrigiert.

**BEISPIEL 1: (+)-[5R(5β,9α,10α)]-2-Chlor-6-methyl-9-phenyl-ergolin**

**a) (-)-(2aS)-1-Benzoyl-4-methylamino-1,2,2a,3-tetrahydrobenz[c,d]indol**

Zu einer Lösung von 20 g (73 mMol) (+)-1-Benzoyl-1,2,2a,3,4,-5-hexahydro-4-ketobenz[c,d]indol und 2 g Monmorillonit (K10)-Katalysator in 200 ml Tetrahydrofuran werden bei 0 - 5 ° 12 g Methylamin eingeleitet. Nach 5 Stunden Rühren bei 0 ° filtriert man die Lösung, dampft das Lösungsmittel ein und kristallisiert aus Methylenchlorid/Ether um. Die so erhaltene Titelverbindung schmilzt bei 163 ° (Zers.). $[\alpha]_D^{20}$ = - 154 ° (c = 1 in Methylenchlorid).

**b) (-)-[3S(9α)]-1-Benzoyl-2,3-dihydro-5,10-didehydro-7-oxo-6-methyl-9-phenyl-ergolin**

Zu einer Lösung von 20,9 g (72 mMol) der unter a) erhaltenen Verbindung und 11 ml (130 mMol) Pyridin in 400 ml Methylenchlorid tropft man bei - 20 ° 12,5 g (75 mMol) Zimtsäurechlorid, gelöst in 60 ml Methylenchlorid. Nach 3 $\frac{1}{2}$ Stunden Nachrühren wird die Reaktionslösung mit Wasser, 2 N Salzsäure und gesättigter $KHCO_3$ gewaschen. Nach Trocknen mit Magnesiumsulfat wird das gelbbraune Oel mit Aceton/Pentan kristallisiert. Smp.: 248 - 250 °. $[\alpha]_D^{20}$ = - 149,3 ° (c = 1 in Methylenchlorid).

**c) (+)-[3S(9α)]-1-Benzyl-2,3-dihydro-5,10-didehydro-6-methyl-9-phenyl-ergolin**

Durch Zutropfen von 10,1 g (100 mMol) Schwefelsäure in eine Suspension von 7,8 g (200 mMol) Lithiumaluminiumhydrid in 175 ml Tetrahydrofuran bei - 30 ° wird Aluminiumhydrid hergestellt. In diese Suspension tropft man bei 15 - 20 ° 17 g (41 mMol) der unter b) erhaltenen Verbindung innert 30 Minuten. Nach 2 Stunden Nachrühren tropft man nacheinander bei 0 ° 25 ml gesättigte $Na_2SO_4$-Lösung und 10 ml konz. NaOH. Nach Filtration und Abdampfen des Lösungsmittels wird der Rückstand aus Ether/Pentan kristallisiert, Smp.: 144 - 146 °. $[\alpha]_D^{20}$ = 111,5 ° (c = 1 in Methylenchlorid).

**d) (-)-[3S(5β,9α,10β)]-1-Benzyl-2,3-dihydro-6-methyl-9-phenylergolin**

In eine Lösung von 14 g (35,6 mMol) der unter c) erhaltenen Verbindung und 50 mg Bromkresol-grün in 450 ml Tetrahydrofuran/Methanol (2 : 1) gibt man bei 0 ° 3,4 g $NaCNBH_3$. Mit 14 ml 4,3 N Salzsäure in

Aethanol wird der pH bei 5 gehalten. Nach 2 ½ Stunden wird die Lösung eingedampft, mit gesättigtem $K_2CO_3$ alkalisch gestellt und mit Methylenchlorid extrahiert. Nach Trocknen und Eindampfen erhält man gelbe Kristalle mit einem Smp. von 89 - 92 °. $[\alpha]_D^{20}$ = - 39,9 ° (c = 1 in Methylenchlorid).

**e) (-)-[3S(5β,9α,10α)]-1-Benzyl-2,3-dihydro-6-methyl-9-phenylergolin**

Eine Lösung von 12 g (32 mMol) der unter, d) erhaltenen Verbindung, 8,5 g (32 mMol) 18-Crown-6 und 3,6 g (32 mMol) Kalium-t.-Butylat in 250 ml abs. Dimethylsulfoxid wird 18 Stunden bei Raumtemperatur gerührt. Nach dem Verdünnen mit 500 ml Eiswasser wird 3 x mit Essigester extrahiert und die organischen Phasen noch 1 x mit Wasser gewaschen, mit Magnesiumsulfat getrocknet und eingedampft. Das dunkelbraune Oel wird aus Aceton/Pentan umkristallisiert. Smp.: 125 - 127 °. $[\alpha]_D^{20}$ = - 107,4 ° (c = 1 in Methylenchlorid).

**f) (-)-[3S(5β,9α,10α)]-2,3-dihydro-6-methyl-9-phenyl-ergolin**

Eine Lösung von 6,0 g (15 mMol) der unter e) erhaltenen Verbindung, 30 ml 1 N Salzsäure und 500 mg Pd-Katalysator (10 %) in 100 ml Methanol wird bei Raumtemperatur hydriert. Nach Filtration des Katalysators wird die Lösung mit 100 ml 2 N Natriumhydroxid versetzt und mit Methylenchlorid extrahiert. Die organischen Phasen werden nach Trocknen mit Magnesiumsulfat eingedampft. Der kristalline Rückstand schmilzt bei 158 - 160 °. $[\alpha]_D^{20}$ = - 86,6 ° (c = 1 in Methylenchlorid).

**g) (+)-[5R(9α,10α)]-6-Methyl-9-phenyl-ergolin**

Eine Lösung von 4,5 g (14,8 mMol) der unter f) erhaltenen Verbindung, 5,2 g (44,3 mMol) Indol und 2,8 g Benzolselensäureanhydrid in 90 ml Tetrahydrofuran wird 4 Stunden bei Raumtemperatur gerührt. Nach Eindampfen kristallisiert man den Rückstand aus Aceton/Ether um. Smp. > 290 °. $[\alpha]_D^{20}$ = 31,0 ° (c = 0,5 in Pyridin).

**h) (+)-[5R(5β,9α,10α)]-2-Chlor-6-methyl-9-phenyl-ergolin**

Zu einer Suspension bestehend aus 2 g (+)-[5R(5β,9α,10α)]-6-Methyl-9-phenyl-ergolin in 30 ml Acetonitril gibt man bei - 10 ° 1,76 ml $BF_3$.Etherat. In die homogene Lösung tropft man innert 5 Minuten bei 0 ° 0,54 ml Sulfurylchlorid. Nach 30 Minuten Rühren wird zur Aufarbeitung mit 5 ml 4 N Natriumcarbonatlösung versetzt, mit Essigester extrahiert, über Natriumsulfat getrocknet, eingedampft und aus Methanol/Aceton umkristallisiert. Smp.: ab 230 ° Zersetzung. $[\alpha]_D^{20}$ = 30,8 ° (c = 0,5 in Pyridin).

Analog zu Beispiel 1 wird folgendes Enantiomer erhalten:

| Beispiel | $R_1$ | $R_2$ | $R_3$ | Smp. (Base) | Drehung |
|---|---|---|---|---|---|
| 2 | $CH_3$ | p-Cl-Phenyl | Cl | 230-245° (Zers.) | + |

Analog zu Beispiel 1, jedoch ausgehend vom (±)-1-Benzoyl-1,2,2a,3,4,5-hexahydro-4-ketobenz[c,d]indol, werden folgende Racemate erhalten:

| Beispiel | $R_1$ | $R_2$ | $R_3$ | Smp. (Base) |
|---|---|---|---|---|
| 3 | $CH_3$ | Phenyl | Cl | 262 - 264 ° |
| 4 | $CH_3$ | p-F-Phenyl | Cl | 251 - 254 ° |
| 5 | $CH_3$ | p-$CF_3$-Phenyl | Cl | 252 - 256 ° (Zers.) |
| 6 | $CH_3$ | Phenyl | Br | 246 - 248 ° |
| 7 | $CH_3$ | Phenyl | J | 223 ° (Zers.) |

**Patentansprüche**

**Patentansprüche für folgende Vertragsstaaten : AT, BE, CH, DE, DK, FR, GB, IT, LI, LU, NL, SE**

1. Eine Verbindung der Formel I,

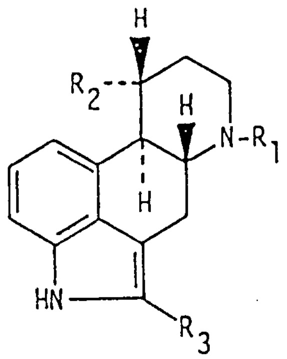

I

worin

$R_1$ Wasserstoff oder Alkyl mit 1 bis 4 Kohlenstoffatomen bedeutet,

$R_2$ für gegebenenfalls durch Fluor, Chlor, Brom, Alkyl oder Alkoxy mit je 1 bis 4 Kohlenstoffatomen oder Trifluoromethyl mono- oder disubstituiertes Phenyl steht und

$R_3$ Chlor, Brom oder Jod bedeutet,

in Form der freien Base oder in Säureadditionssalzform.

2. Verbindung der Formel I gemäss Anspruch 1, worin $R_1$, $R_2$ und $R_3$ beziehungsweise
   $CH_3$, p-Cl-Phenyl und Cl;
   $CH_3$, Phenyl und Cl;
   $CH_3$, p-F-Phenyl und Cl;
   $CH_3$, p-$CF_3$-Phenyl und Cl;
   $CH_3$, Phenyl und Br; oder
   $CH_3$, Phenyl und J
   bedeuten, in Form der freien Base oder in Säureadditionssalzform.

3. Das (+)-[5R(5$\beta$,9$\alpha$,10$\alpha$)]-2-Chlor-6-methyl-9-phenyl-ergolin in Form der freien Base oder in Säureadditionssalzform.

4. Verfahren zur Herstellung einer Verbindung gemäss Anspruch 1, dadurch gekennzeichnet, dass man Verbindungen der Formel II,

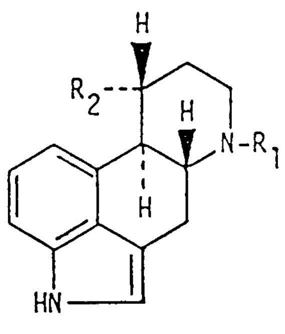

II

worin $R_1$ und $R_2$ wie im Anspruch 1 definiert sind, halogeniert und die erhaltenen Verbindungen der Formel I in Form der freien Basen oder in Säureadditionssalzform gewinnt.

5. Eine Verbindung gemäss einem der Ansprüche 1 bis 3, in freier oder physiologisch verträglicher Säureadditionssalzform, zur Anwendung als Pharmazeutikum.

6. Eine Verbindung gemäss einem der Ansprüche 1 bis 3, in freier oder physiologisch verträglicher Säureadditionssalzform, zur Behandlung der Schizophrenie.

7. Eine pharmazeutische Zusammensetzung, enthaltend eine Verbindung gemäss einem der Ansprüche 1 bis 3, in freier oder physiologisch verträglicher Säureadditionssalzform.

8. Die Anwendung einer Verbindung gemäss einem der Ansprüche 1 bis 3, für die Herstellung einer pharmazeutischen Zusammensetzung zur Behandlung der Schizophrenie.

**Patentansprüche für folgende Vertragsstaaten : ES, GR**

1. Verfahren zur Herstellung von Verbindungen der Formel I,

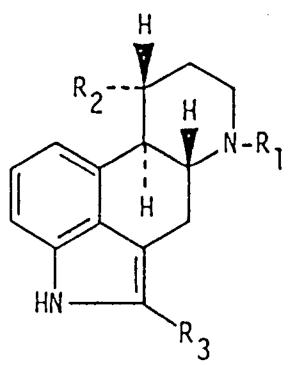

I

worin

$R_1$ Wasserstoff oder Alkyl mit 1 bis 4 Kohlenstoffatomen bedeutet,

$R_2$ für gegebenenfalls durch Fluor, Chlor, Brom, Alkyl oder Alkoxy mit je 1 bis 4 Kohlenstoffatomen oder Trifluoromethyl mono- oder disubstituiertes Phenyl steht und

$R_3$ Chlor, Brom oder Jod bedeutet,

und ihren Säureadditionssalzen, dadurch gekennzeichnet, dass man Verbindungen der Formel II,

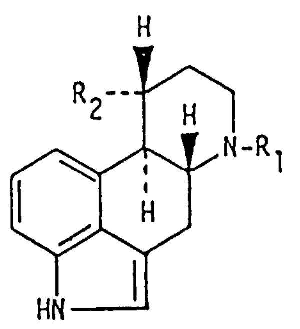

II

worin $R_1$ und $R_2$ obige Bedeutung besitzen, halogeniert und die erhaltenen Verbindungen der Formel I in Form der freien Basen oder in Säureadditionssalzform gewinnt.

2. Verfahren gemäss Anspruch 1, dadurch gekennzeichnet, dass man eine Verbindung der Formel I herstellt, worin $R_1$, $R_2$ und $R_3$ beziehungsweise

$CH_3$, p-Cl-Phenyl und Cl;
$CH_3$, Phenyl und Cl;
$CH_3$, p-F-Phenyl und Cl;
$CH_3$, p-$CF_3$-Phenyl und Cl;
$CH_3$, Phenyl und Br; oder
$CH_3$, Phenyl und J

bedeuten.

3. Verfahren gemäss Anspruch 1, zur Herstellung des (+)-[5R(5β,9α,10α)]-2-Chlor-6-methyl-9-phenyl-ergolins in Form der freien Base oder eines Säureadditionssalzes.

4. Verfahren zur Herstellung einer pharmazeutischen Zusammensetzung, enthaltend eine Verbindung der Formel I gemäss Anspruch 1 in Form der freien Base oder eines physiologisch verträglichen Säureadditionssalzes, dadurch gekennzeichnet, dass man die Verbindung der Formel I mit einem pharmazeutischen Hilfs- oder Trägerstoff vermischt.

**Claims**

**Claims for the following Contracting States : AT, BE, CH, DE, DK, FR, GB, IT, LI, LU, NL, SE**

1. A compound of formula I,

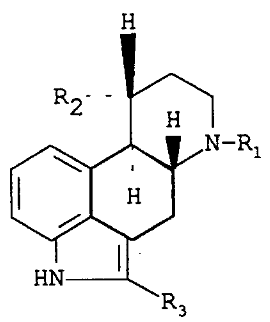

I

wherein

$R_1$ signifies hydrogen or alkyl with 1 to 4 carbon atoms,

$R_2$ is phenyl which is optionally mono- or di-substituted by fluorine, chlorine, bromine, alkyl or alkoxy each with 1 to 4 carbon atoms, or by trifluoromethyl, and

$R_3$ signifies chlorine, bromine or iodine,

in free base form or in acid addition salt form.

2. Compound of formula I according to claim 1, wherein $R_1$, $R_2$ and $R_3$ respectively signify

$CH_3$, p-Cl-phenyl and Cl;
$CH_3$, phenyl and Cl;
$CH_3$, p-F-phenyl and Cl;
$CH_3$, p-$CF_3$-phenyl and Cl;
$CH_3$, phenyl and Br; or
$CH_3$, phenyl and I,

in free base form or in acid addition salt form.

3. (+)-[5R(5β,9α,10α)]-2-chloro-6-methyl-9-phenyl-ergoline in free base form or in acid addition salt form.

4. Process for the production of a compound according to claim 1, characterized in that compounds of formula II,

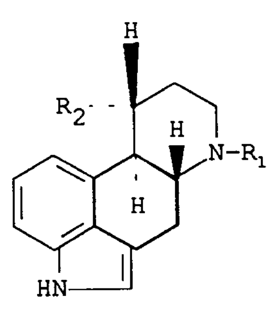

II

wherein $R_1$ and $R_2$ are defined as in claim 1, are halogenated, and the compounds of formula I thus

obtained are recovered in free base form or in acid addition salt form.

**5.** A compound according to one of claims 1 to 3, in free form or in physiologically tolerable acid addition salt form, for use as a pharmaceutical.

**6.** A compound according to one of claims 1 to 3, in free form or in physiologically tolerable acid addition salt form, for the treatment of schizophrenia.

**7.** A pharmaceutical composition, containing a compound according to one of claims 1 to 3, in free form or in physiologically tolerable acid addition salt form.

**8.** Use of a compound according to one of claims 1 to 3, in the production of a pharmaceutical composition for the treatment of schizophrenia.

**Claims for the following Contracting States : ES, GR**

**1.** Process for the production of compounds of formula I,

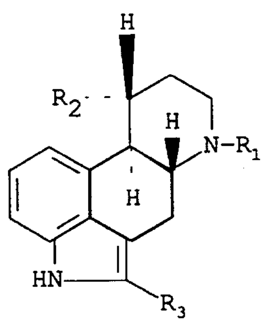

I

wherein
- $R_1$ signifies hydrogen or alkyl with 1 to 4 carbon atoms,
- $R_2$ is phenyl which is optionally mono- or di-substituted by fluorine, chlorine, bromine, alkyl or alkoxy each with 1 to 4 carbon atoms, or by trifluoromethyl, and
- $R_3$ signifies chlorine, bromine or iodine,

and their acid addition salts, characterized in that compounds of formula II,

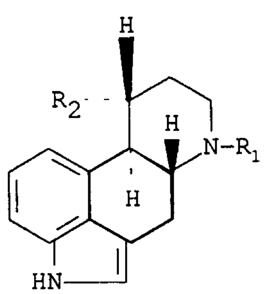

II

wherein $R_1$ and $R_2$ are defined as above, are halogenated,
and the compounds of formula I thus obtained are recovered in free base form or in acid addition salt form.

**2.** Process according to claim 1, characterized in that a compound of formula I is produced, in which $R_1$, $R_2$ and $R_3$ respectively signify
$CH_3$, p-Cl-phenyl and Cl;
$CH_3$, phenyl and Cl;
$CH_3$, p-F-phenyl and Cl;
$CH_3$, p-$CF_3$-phenyl and Cl;

$CH_3$, phenyl and Br; or
$CH_3$, phenyl and I.

**3.** Process according to claim 1, for the production of (+)-[5R(5β,9α,10α)]-2-chloro-6-methyl-9-phenyl-ergoline in free base form or in acid addition salt form.

**4.** Process for the production of a pharmaceutical composition, containing a compound of formula I according to claim 1, in free base form or in the form of a physiologically tolerable acid addition salt, characterized in that the compound of formula I is mixed with a pharmaceutical assistant or carrier material.

**Revendications**

**Revendications pour les Etats contractants suivants : AT, BE, CH, DE, DK, FR, GB, IT, LI, LU, NL, SE**

**1.** Un composé de formule I

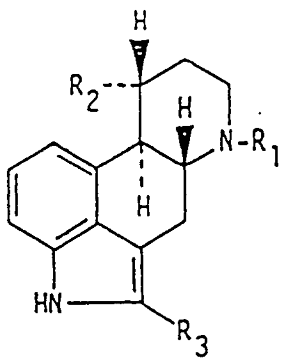

I

dans laquelle

- $R_1$ signifie l'hydrogène ou un groupe alkyle contenant de 1 à 4 atomes de carbone,
- $R_2$ signifie un groupe phényle éventuellement mono- ou di-substitué par le fluor, le chlore ou le brome ou par des groupes alkyle ou alcoxy contenant chacun de 1 à 4 atomes de carbone ou trifluorométhyle, et
- $R_3$ signifie le chlore, le brome ou l'iode,

sous forme de base libre ou d'un sel d'addition d'acide.

**2.** Un composé de formule I selon la revendication 1, dans lequel $R_1$, $R_2$ et $R_3$ signifient respectivement
$CH_3$, p-Cl-phényl et Cl;
$CH_3$, phényl et Cl;
$CH_3$, p-F-phényl et Cl;
$CH_3$, p-$CF_3$-phényl et Cl;
$CH_3$, phényl et Br; ou
$CH_3$, phényl et I,
sous forme de base libre ou d'un sel d'addition d'acide.

**3.** La(+)-[5R(5β,9α,10α)]-2-chloro-6-méthyl-9-phényl-ergoline sous forme de base libre ou d'un sel d'addition d'acide.

**4.** Procédé de préparation d'un composé selon la revendication 1, caractérisé en ce qu'on halogène des composés de formule II

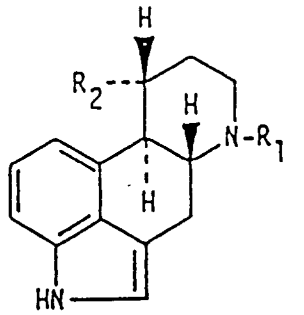

II

dans laquelle $R_1$ et $R_2$ sont tels que définis à la revendication 1, et on récupère les composés obtenus de formule I sous forme de bases libres ou de sels d'addition d'acides.

**5.** Un composé selon l'une quelconque des revendications 1 à 3, sous forme libre ou sous forme d'un sel d'addition d' acide physiologiquement acceptable, pour l'utilisation comme médicament.

**6.** Un composé selon l'une quelconque des revendications 1 à 3, sous forme libre ou sous forme d'un sel d'addition d' acide physiologiquement acceptable, pour le traitement de la schizophrénie.

**7.** Une composition pharmaceutique, contenant un composé selon l'une quelconque des revendications 1 à 3, sous forme libre ou sous forme d'un sel d'addition d'acide physiologiquement acceptable.

**8.** L'utilisation d'un composé selon l'une quelconque des revendications 1 à 3, pour la préparation d'une composition pharmaceutique destinée au traitement de la schizophrénie.

**Revendications pour les Etats contractants suivants : ES, GR**

**1.** Procédé de préparation des composés de formule I

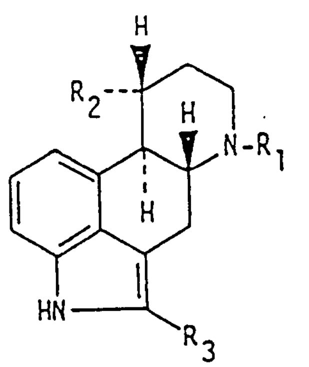

I

dans laquelle

- $R_1$ signifie l'hydrogène ou un groupe alkyle contenant de 1 à 4 atomes de carbone,
- $R_2$ signifie un groupe phényle éventuellement mono- ou di-substitué par le fluor, le chlore ou le brome ou par des groupes alkyle ou alcoxy contenant chacun de 1 à 4 atomes de carbone ou trifluorométhyle, et
- $R_3$ signifie le chlore, le brome ou l'iode,

et de leurs sels d'addition d'acides, caractérisé en ce qu'on halogène des composés de formule II

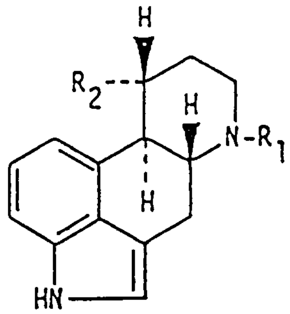

II

dans laquelle $R_1$ et $R_2$ possèdent les significations données ci-dessus, et on récupère les composés obtenus de formule I sous forme de bases libres ou sous forme de sels d'addition d'acides.

**2.** Un procédé selon la revendication 1, caractérisé en ce qu'on prépare un composé de formule I dans laquelle $R_1$, $R_2$ et $R_3$ signifient respectivement
$CH_3$, p-Cl-phényl et Cl;
$CH_3$, phényl et Cl;
$CH_3$, p-F-phényl et Cl;
$CH_3$, p-$CF_3$-phényl et Cl;
$CH_3$, phényl et Br; ou
$CH_3$, phényl et I.

**3.** Un procédé selon la revendication 1, pour la préparation de la (+)-[5R(5β,9α,10α)]-2-chloro-6-méthyl-9-phényl-ergoline sous forme de base libre ou d'un sel d'addition d'acide.

**4.** Procédé de préparation d'une composition pharmaceutique, contenant un composé de formule I selon la revendication 1 sous forme de base libre ou d'un sel d'addition d'acide physiologiquement acceptable, caractérisé en ce qu'on mélange le composé de formule I avec un support ou excipient pharmaceutique.